# EUROPEAN PATENT SPECIFICATION

(11) **EP 4 117 595 B1**
(45) Date of publication and mention of the grant of the patent: **18.11.2026**
(21) Application number: 21720056.7
(22) Date of filing: 19.03.2021
(51) Int. Cl.: A61G 13/10, A61B 90/40

(54) **ENCLOSURES INCLUDING SYSTEMS AND METHODS THEREOF FOR ESTABLISHING A STERILE ENVIRONMENT FOR A MEDICAL PROCEDURE**
GEHÄUSE MIT SYSTEMEN UND VERFAHREN DAFÜR ZUR HERSTELLUNG EINER STERILEN UMGEBUNG FÜR EINEN MEDIZINISCHEN EINGRIFF
ENCEINTES COMPRENANT DES SYSTÈMES ET PROCÉDÉS ASSOCIÉS POUR ÉTABLIR UN ENVIRONNEMENT STÉRILE POUR UNE INTERVENTION MÉDICALE

(30) Priority: 19.03.2020 US 202062992062 P
(43) Date of publication of application: 18.01.2023
(73) Proprietor: Bard Access Systems, Inc., Salt Lake City, UT 84116 (US)
(72) Inventor: TA, Teresa, Murray, UT 84107 (US); DAVIS, Michael, West Jordan, UT 84081 (US); THORNLEY, Kyle, G., Farmington, UT 84025 (US); BEAL, James, D., South Jordan, UT 84095 (US); BLANCHANRD, Daniel, B., Bountiful, UT 84010 (US); HOWELL, Glade, H., Draper, UT 84020 (US)
(74) Representative: Hoffmann Eitle
(86) International application number: PCT/US2021/023268
(87) International publication number: WO 2021/188970

(56) References cited:
- WO-A1-2004/024014
- WO-A1-2014/145032
- WO-A1-2014/189874
- WO-A1-94/20041
- US-A- 4 612 916
- US-A- 5 316 541
- US-A1- 2002 045 796

## Description

### BACKGROUND

Medical procedures such as surgical procedures require taking certain precautions to ensure the immediate environment in which the medical procedures are performed is sterile. Ensuring such an environment is sterile protects patients from infection when their natural protective barrier (i.e., their skin) is broken for the medical procedures.

WO 2014/189874 A1, US 5,316,541, WO 2014/145032 A1 and US 2002/045796 A1 relate to sterile enclosures for performing medical procedures. WO 94/20041 A1 relates to a wound treatment apparatus or applying negative pressure to a sound.

Disclosed herein are enclosures for establishing a sterile environment over a patient for a medical procedure, as well as systems thereof.

### SUMMARY

Disclosed herein is an enclosure for establishing a sterile environment over a patient for a medical procedure. The enclosure includes an expandable portion of a barrier and a patient-interfacing portion of the barrier. The expandable portion of the barrier separates the sterile environment inside of the enclosure from a non-sterile environment outside of the enclosure. The expandable portion of the barrier is configured to expand from an unexpanded state to an expanded state for working within the sterile environment. The patient-interfacing portion of the barrier includes a fenestration configured for placement directly over an area of interest of a patient. The fenestration has a sealed state and an unsealed state for access to the area of interest from within the sterile environment.

The enclosure further includes a support system coupled to the patient-interfacing portion of the barrier flanking the fenestration. The support system is configured to support the enclosure on a surface when placed over an appendage of the patient or a main body of the patient. The patient can be positioned in any one of a number of anatomical positions on the surface.

In some embodiments, the support system is configured to expand from an unexpanded state to an expanded state in concert with the expandable portion of the barrier.

In some embodiments, the support system is configured to expand from an unexpanded state to an expanded state independent of the expandable portion of the barrier.

In some embodiments, the enclosure further includes a pair of framed ends of the enclosure configured to support the expandable portion of the barrier. Each framed end of the pair of framed ends is configured to lie down on the patient-interfacing portion of the barrier in the unexpanded state of the barrier. Each framed end of the pair of framed ends is also configured to extend vertically from the patient-interfacing portion of the barrier in the expanded state of the barrier.

In some embodiments, the enclosure further includes an air inlet fluidly connected to the inside of the enclosure. The air inlet is configured for introducing filtered air into the inside of the enclosure for inflating the expandable portion of the barrier from the unexpanded state to the expanded state or maintaining a greater pressure inside of the enclosure than outside of the enclosure.

In some embodiments, the enclosure further includes an inline high-efficiency particulate-arresting ("HEPA") air filter optionally including an antimicrobial coating on a filter medium of the HEPA air filter. The HEPA air filter is configured to prevent microbial contamination of the sterile environment inside of the enclosure.

In some embodiments, the fenestration includes an outer seal over a patient-interfacing surface of the patient-interfacing portion of the barrier in the sealed state of the fenestration.

In some embodiments, a perimeter of the patient-interfacing surface of the patient-interfacing portion of the barrier around the fenestration includes an adhesive. The adhesive is configured to adhere the patient-interfacing portion of the barrier to the patient with the perimeter of the fenestration around the area of interest subsequent to sterilization thereof. The outer seal of the fenestration is configured to protect and maintain an integrity of the adhesive prior to use.

In some embodiments, the fenestration includes an inner seal over an inner surface of the patient-interfacing portion of the barrier in the sealed state of the fenestration. The inner seal of the fenestration is configured to be removed subsequent to adhering the patient-interfacing portion of the barrier to the patient. Removing the inner seal subsequent to adhering the patient-interfacing portion of the barrier to the patient maintains the sterile environment over the patient.

In some embodiments, the enclosure further includes one or more pairs of long-sleeved gloves integrated into the expandable portion of the barrier. The long-sleeved gloves are configured to be worn by a clinician when performing the medical procedure within the sterile environment.

In some embodiments, the enclosure further includes one or more closed-ended tubes integrated into the expandable portion of the barrier. The one-or-more closed-ended tubes are configured to accept insertion of a probe for use during the medical procedure within the sterile environment.

In some embodiments, the enclosure further includes one or more pockets integrated into the expandable portion of the barrier. The one-or-more pockets are configured to hold procedural equipment or supplies for the medical procedure. The one-or-more pockets are inside of the enclosure, outside of the enclosure, or a combination thereof.

In some embodiments, the enclosure further includes an introducing means for introducing additional procedural equipment or supplies into the sterile environment inside of the enclosure from the non-sterile environment outside of the enclosure.

Also disclosed herein is a system for establishing a sterile environment over a patient for a medical procedure. The system includes an enclosure for placement over a patient and procedural equipment or supplies for the medical procedure in a sterile environment inside of the enclosure. The enclosure includes an expandable portion of a barrier and a patient-interfacing portion of the barrier. The expandable portion of the barrier separates the sterile environment inside of the enclosure from a non-sterile environment outside of the enclosure. The expandable portion of the barrier is configured to expand from an unexpanded state to an expanded state for working within the sterile environment. The patient-interfacing portion of the barrier includes a fenestration configured for placement directly over an area of interest of a patient. The fenestration has a sealed state and an unsealed state for access to the area of interest from within the sterile environment.

The enclosure further includes a support system coupled to the patient-interfacing portion of the barrier flanking the fenestration. The support system is configured to support the enclosure on a surface when placed over an appendage of the patient or a main body of the patient. The patient can be positioned in any one of a number of anatomical positions on the surface.

In some embodiments, the fenestration includes an outer seal over a patient-interfacing surface of the patient-interfacing portion of the barrier in the sealed state of the fenestration.

In some embodiments, a perimeter of the patient-interfacing surface of the patient-interfacing portion of the barrier around the fenestration includes an adhesive. The adhesive is configured to adhere the patient-interfacing portion of the barrier to the patient with the perimeter of the fenestration around the area of interest subsequent to sterilization thereof. The outer seal of the fenestration is configured to protect and maintain an integrity of the adhesive prior to use.

In some embodiments, the fenestration includes an inner seal over an inner surface of the patient-interfacing portion of the barrier in the sealed state of the fenestration. The inner seal of the fenestration is configured to be removed subsequent to adhering the patient-interfacing portion of the barrier to the patient. Removing the inner seal subsequent to adhering the patient-interfacing portion of the barrier to the patient maintains the sterile environment over the patient.

In some embodiments, the procedural equipment includes one or more puncturing devices, one or more guidewires selected from access guidewires, maneuver guidewires, and rail guidewires, one or more catheters, one or more syringes, or a combination thereof.

In some embodiments, the supplies include one or packages of an antiseptic, saline, or a medicament, one or more swabs, gauze, one or more drapes, or a combination thereof.

These and other features of the concepts provided herein will become more apparent to those of skill in the art in view of the accompanying drawings and following description, which describe particular embodiments of such concepts in greater detail.

### DRAWINGS

FIG. 1 illustrates a system including an enclosure for establishing a sterile environment in accordance with some embodiments.
FIG. 2 illustrates the system of FIG. 1 including an enclosure with a barrier-crossing tube in accordance with some embodiments.
FIG. 3 illustrates the system of FIG. 1 including an enclosure with a door in accordance with some embodiments.
FIG. 4 illustrates the system of FIG. 1 including an enclosure with a chute in accordance with some embodiments.
FIG. 5 illustrates an alternative enclosure for the system of FIG. 1 in accordance with some embodiments.
FIG. 6 illustrates another alternative enclosure for the system of FIG. 1 in accordance with some embodiments.

### DESCRIPTION

The invention is defined in appended independent claims 1 and 9. Further embodiments of the invention are defined in appended dependent claims.

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

### Enclosures for establishing a sterile environment

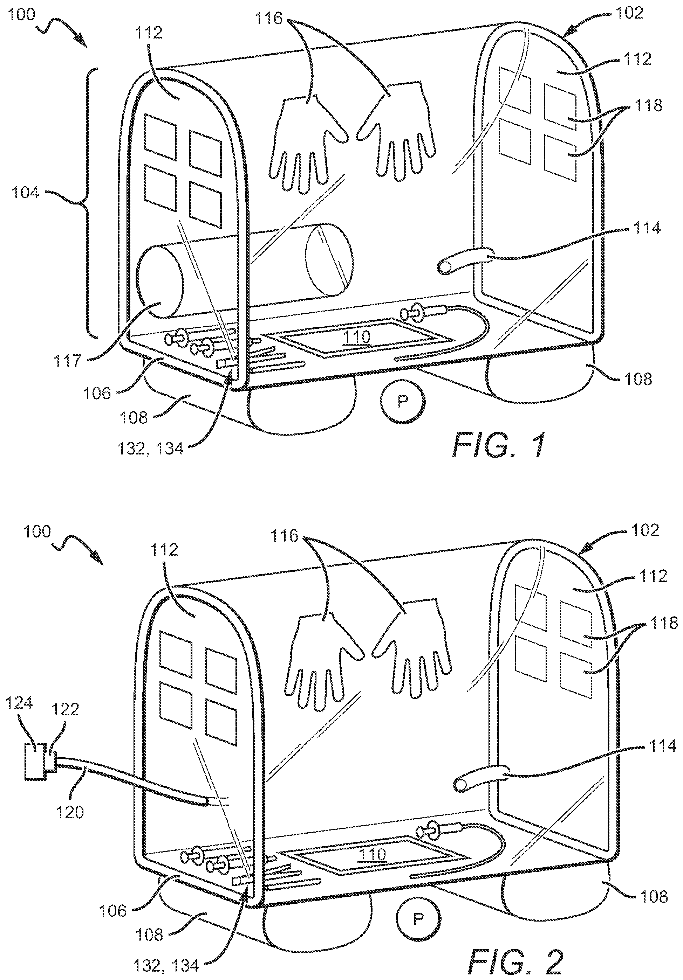

FIG. 1 illustrates a system 100 including an enclosure 102 for establishing a sterile environment in accordance with some embodiments. While FIG. 1 shows a system including the enclosure 102 for establishing a sterile environment, the enclosure 102 will be primarily described in this section. Systems for establishing a sterile environment are primarily described in more detail below in the section on such systems.

The enclosure 102 is configured for establishing a sterile environment over a patient for a medical procedure. The enclosure 102 can be of any size suitable for the medical procedure.

As shown, the enclosure 102 includes a barrier formed of an expandable portion 104 of the barrier and a patient-interfacing portion 106 of the barrier. The barrier separates the sterile environment inside of the enclosure 102 from a non-sterile environment outside of the enclosure 102. As such, each portion of the expandable portion 104 and the patient-interfacing portion 106 of the barrier separates the sterile environment inside of the enclosure 102 from a non-sterile environment outside of the enclosure 102.

The expandable portion 104 of the barrier is configured to expand from an unexpanded state of the expandable portion 104 to an expanded state for working within the sterile environment. The expandable portion 104 can be an inflatable bag of a translucent plastic (e.g., polyethylene, styrene, polycarbonate, etc.) over the patient-interfacing portion 106 of the barrier or a framed structure including the translucent plastic stretched between frame elements.

With respect to the framed structure, for example, the framed structure can include a pair of framed ends 112 of the enclosure 102 configured to support the expandable portion 104 of the barrier. Each framed end of the pair of framed ends 112 is configured to lie down on the patient-interfacing portion 106 of the barrier in the unexpanded state of the barrier. Each framed end of the pair of framed ends 112 is also configured to extend vertically from the patient-interfacing portion 106 of the barrier in the expanded state of the barrier. Each framed end of the pair of framed ends can be independently lifted from the patient-interfacing portion 106 of the barrier in the unexpanded state of the expandable portion 104 of the barrier to form the expanded state thereof with simultaneous introduction of filtered air commensurate with a rate at which the pair of framed ends are lifted from the patient-interfacing portion 106 of the barrier. Alternatively, the framed structure is inflated by introduction of the filtered air.

In view of the foregoing, the enclosure 102 can further include an air inlet 114 fluidly connected to the inside of the enclosure 102. The air inlet 114 is configured for introducing air (e.g., filtered air) into the inside of the enclosure 102 for expanding the enclosure 102 from the unexpanded state of the expandable portion 104 to the expanded state thereof. For example, the air inlet is configured for equilibrating pressure inside of the enclosure 102 when expanding the expandable portion 104 of the framed structure set forth above from the unexpanded state to the expanded state thereof. And, for example, the air inlet is configured for inflating the expandable portion 104 of the inflatable bag set forth above from the unexpanded state to the expanded state thereof. In addition, the air inlet 114 is configured for introducing air (e.g., filtered air) into the inside of the enclosure 102 for maintaining a greater pressure inside of the enclosure 102 than outside of the enclosure 102 when needed. The air inlet 114 can be fluidly connected to a pump for inflating the expandable portion 104 of the barrier or maintaining the greater pressure inside of the enclosure 102 than outside of the enclosure 102.

It should be understood that whether the expandable portion 104 of the barrier includes the inflatable bag or the framed structure, the expandable portion 104 of the barrier can be inflated through the air inlet 114. In addition, maintaining a greater pressure inside of the enclosure 102 than outside of the enclosure 102 is particularly important in maintaining the structural integrity of the expandable portion 104 of the barrier when the expandable portion 104 is the inflatable bag.

To provide filtered air into the inside of the enclosure 102, the enclosure can further include an inline HEPA air filter optionally including an antimicrobial coating on a filter medium of the HEPA air filter. The HEPA air filter is configured to prevent microbial contamination of the sterile environment inside of the enclosure 102.

The patient-interfacing portion 106 of the barrier includes a fenestration 110 configured for placement directly over an area of interest (e.g., surgical area, wound such as an open wound, a previously closed and dressed wound, a burn wound, etc.) of a patient P; however, the patient-interfacing portion 106 of the barrier is not limited to the fenestration 110. Indeed, the patient-interfacing portion 106 of the barrier can include any number of additional fenestrations to the fenestration 110. The fenestration 110 has a sealed state and an unsealed state for access to the area of interest from within the sterile environment.

The fenestration 110 includes an outer seal over a patient-interfacing surface (e.g., outer surface) of the patient-interfacing portion 106 of the barrier in the sealed state of the fenestration 110. A perimeter of the patient-interfacing surface around the fenestration 110 includes an adhesive configured to adhere the patient-interfacing portion 106 of the barrier to the patient P with the perimeter of the fenestration 110 around the area of interest subsequent to sterilization thereof. The outer seal of the fenestration 110 is configured to protect and maintain an integrity of the adhesive prior to use.

The fenestration 110 also includes an inner seal over an inner surface of the patient-interfacing portion 106 of the barrier in the sealed state of the fenestration 110. The inner seal of the fenestration 110 is configured to be removed subsequent to adhering the patient-interfacing portion 106 of the barrier to the patient P. Removing the inner seal subsequent to adhering the patient-interfacing portion 106 of the barrier to the patient maintains the sterile environment over the patientP by stopping unfiltered air from entering the enclosure 102 through the fenestration 110.

As shown, the enclosure 102 further includes a support system 108 coupled to the patient-interfacing portion 106 of the barrier. When present, the support system 108 is coupled to the patient-interfacing portion 106 of the barrier and includes at least a pair of supports flanking the fenestration 110. The support system 108 is configured to support the enclosure 102 on a surface (e.g., a top of an operating table, a top of a bed, etc.) when placed over an appendage of the patient P or a main body of the patient P such that the supports flank the appendage or main body of the patient P. The patient P can be positioned in any one of a number of anatomical positions (e.g., supine, prone, right lateral recumbent, left lateral recumbent, etc.) on the surface for use with the enclosure 102.

The support system 108 can be immediately ready to be placed over the patient P subsequent to unpackaging the enclosure 102 or the support system 108 can be configured to expand from an unexpanded state to an expanded state for use with the patient P. With respect to the former, for example, the supports of support system 108 can include fiberfill stuffing, thereby making the support system 108 immediately ready to be placed over the patient subsequent to unpackaging. With respect to the latter, for example, the support system 108 can be configured to expand from the unexpanded state to the expanded state in concert with the expandable portion 104 of the barrier. That is, the supports of the support system 108 can be fluidly connected to the inside of the enclosure 102 such that when the expandable portion 104 of the barrier is inflated, the support system 108 is also inflated. Alternatively, the support system 108 can be configured to expand from the unexpanded state to the expanded state independent of the expandable portion 104 of the barrier through one or more inlets exclusive to the support system 108. In this way, the supports of the support system 108 can be expanded from the unexpanded state to the expanded state with a different fluid than the expandable portion 104 of the barrier. Instead of air, for example, water can be used to expand the support system 108 from the unexpanded state to the expanded state. Water is advantageous in that it provides more weight to the enclosure 102 than air and lowers a center of gravity of the enclosure 102 as well.

The enclosure 102 can further include one or more pairs of long-sleeved gloves 116 integrated into the translucent plastic of the expandable portion 104 of the barrier. The one-or-more pairs of long-sleeved gloves 116 are configured to be worn by a clinician when performing the medical procedure within the sterile environment of the enclosure 102.

The enclosure 102 can further include one or more closed-ended tubes 117 integrated into translucent plastic of the expandable portion of the barrier. Instead of being configured with fingertips like the one-or-more long-sleeved gloves, the one-or-more closed-ended tubes 117 are configured to accept insertion of a probe (e.g., an ultrasound probe) for use during the medical procedure within the sterile environment of the enclosure 102.

The enclosure 102 can further include one or more pockets 118 integrated into the expandable portion 104 or the patient-facing portion 106 of the barrier. When present, the one-or-more pockets 118 are configured to hold procedural equipment or supplies for the medical procedure. The one-or-more pockets 118 are also configured to hold the procedural equipment or supplies subsequent to the use thereof during the medical procedure, thereby safely keeping biohazardous waste safely contained in the enclosure 102. The one-or-more pockets 118 can be inside of the enclosure 102, outside of the enclosure 102, or a combination thereof. Additionally or alternatively, the enclosure 102 can include loops, clamps, hook-and-loop fasteners, snaps, glue dots, or other fastening devices to hold the procedural equipment or supplies for the medical procedure. The enclosure 102 can further include one or more rigid trays configured to hold the procedural equipment or supplies for the medical procedure. Such rigid trays can include any of the foregoing fastening devices for holding the procedural equipment, and the enclosure 102, in turn, can include any of the foregoing fastening devices for holding the rigid trays.

The enclosure 102 can further include an introducing means for introducing procedural equipment or supplies into the sterile environment inside of the enclosure 102 from the non-sterile environment outside of the enclosure.

FIG. 2 illustrates the system 100 of FIG. 1 including the enclosure 102 with a barrier-crossing tube 120 in accordance with some embodiments. While the tube 120 is shown crossing the barrier of a framed end of the pair of framed ends 112 of the enclosure 102, the tube 120 can cross any portion of the barrier that separates the sterile environment inside of the enclosure 102 from a non-sterile environment outside of the enclosure 102. The tube 120 can include a Luer connector 122 and a cap 124 configured to maintain a sterility of a lumen of the tube 120 for introducing supplies into the sterile environment inside of the enclosure 102 such as a guidewire, saline, a solution including a medicament, or the like.

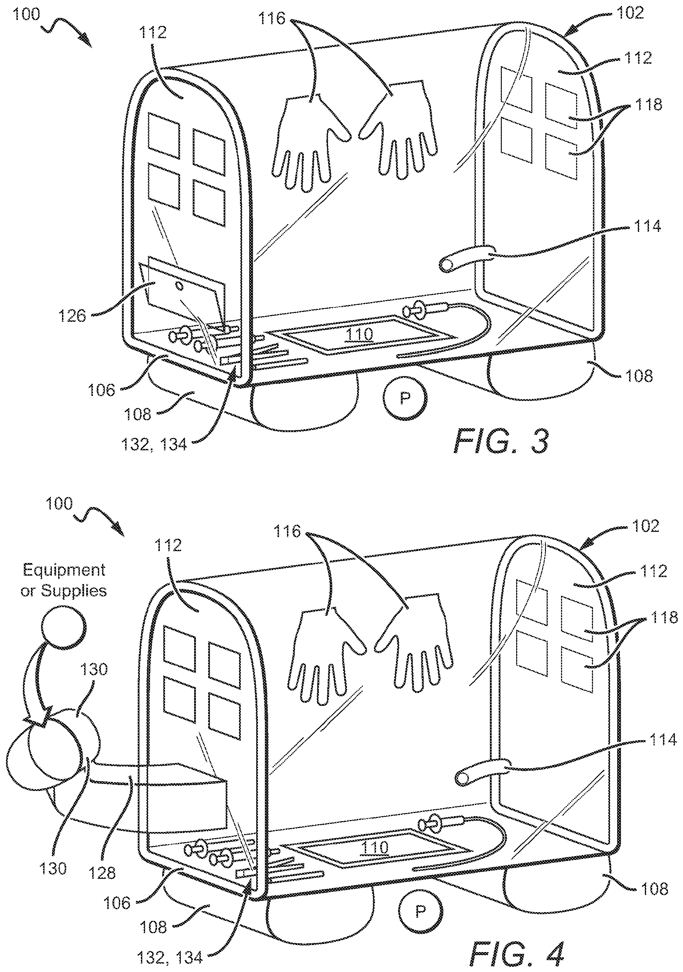

FIG. 3 illustrates the system 100 of FIG. 1 including the enclosure 102 with a door 126 in accordance with some embodiments. While the door 126 is shown in the barrier of a framed end of the pair of framed ends 112 of the enclosure 102, the door 126 can be in any portion of the barrier that separates the sterile environment inside of the enclosure 102 from a non-sterile environment outside of the enclosure 102. The door 126 can include a knob, hook, or the like on one side or both sides of the door 126 configured to facilitate opening the door 126 for introducing the procedural equipment or supplies into the sterile environment inside of the enclosure 102 and closing the door 126 thereafter.

FIG. 4 illustrates the system 100 of FIG. 1 including the enclosure 102 with a chute 128 in accordance with some embodiments. While the chute 128 is shown extending from the barrier of a framed end of the pair of framed ends 112 of the enclosure 102, the chute 128 can be extend from any portion of the barrier that separates the sterile environment inside of the enclosure 102 from a non-sterile environment outside of the enclosure 102. The chute 128 can include a fold-top end having one or more flaps 130 configured to fold over the chute 128 in a closed state of the chute 128. The one-or-more flaps 130 are configured to open to one or more sides of the chute 128 for an open state of the chute 128 for introducing the procedural equipment or supplies into the sterile environment inside of the enclosure 102. The one-or-more flaps 130 can include a sterile side corresponding to an inside of the chute 128 when in the closed state thereof. Handling the one-or-more flaps 130 only by a non-sterile side of the one-or-more flaps 130 opposite the sterile side ensures the procedural equipment or supplies dropped into the chute 128 do not come into contact with a non-sterile surface such as the non-sterile side of the one-or-more flaps 130.

The chute 128 can be formed from any tube of the one-or-more closed-ended tubes set forth above configured to accept insertion of a probe (e.g., an ultrasound probe) for use during the medical procedure within the sterile environment of the enclosure 102. Indeed, such closed-ended tubes can have scores or similar weakened areas that can be broken apart to create the fold-top end having the one-or-more flaps 130.

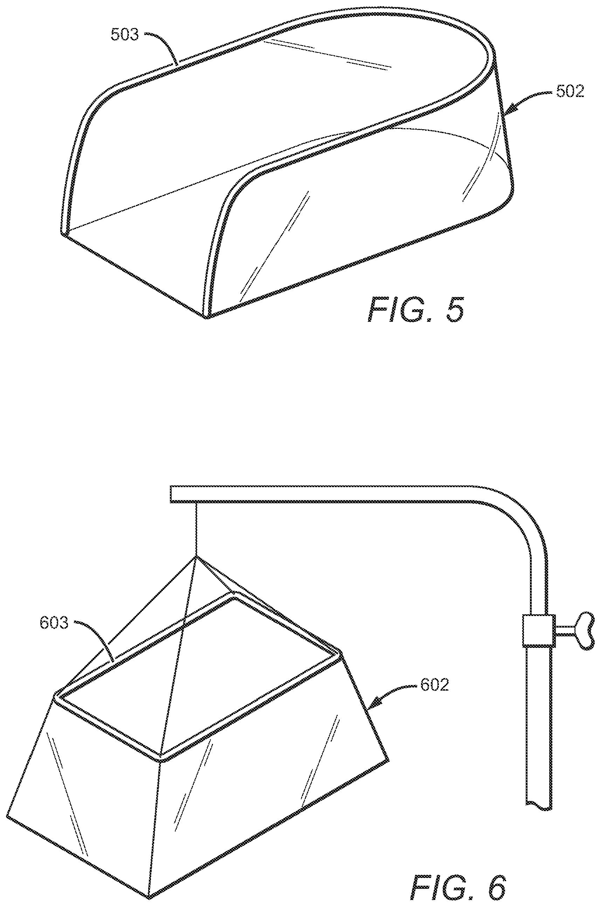

FIG. 5 illustrates an alternative enclosure 502 for the system 100 of FIG. 1 in accordance with some embodiments. Like the enclosure 102 of any of FIGS. 1-4, the enclosure 502 is configured for establishing a sterile environment over a patient for a medical procedure. The enclosure 502 is a framed structure like that set forth above for the enclosure 102; however, the enclosure 502 includes a pop-up frame 503 including the translucent plastic stretched between portions of the pop-up frame 503.

The enclosure 502 is configured with an unexpanded state in which the pop-up frame 503 is twisted and folded upon itself in one or more revolutions of the pop-up frame 503, thereby locking an amount of torsional potential energy into the pop-up frame 503 in the unexpanded state of the enclosure 502. The enclosure 502 is also configured with an expanded state in which the pop-up frame 503 is untwisted and unfolded. A transition from the unexpanded state to the expanded state of the enclosure 502 is effectuated by releasing the torsional potential energy in the pop-up frame 503 by perturbing an energetic state of the pop-up frame 503 at a local minimum in the unexpanded state or by unclipping one or more clips that clip together the one-or-more revolutions of the pop-up frame 503 in the unexpanded state.

While not shown, the enclosure 502 includes one or more features set forth above for the enclosure 102 selected from at least the air inlet 114, the fenestration 110, the one-or-more pairs of long-sleeved gloves 116, the one-or-more closed-ended tubes 117, the one-or-more pockets 118, and the introducing means for introducing procedural equipment or supplies into the sterile environment inside of the enclosure 502.

FIG. 6 illustrates the system of FIG. 1 with another alternative enclosure in accordance with some embodiments.

Like the enclosure 102 of any figure of FIGS. 1-4, the enclosure 602 is configured for establishing a sterile environment over a patient for a medical procedure. The enclosure 602 is a framed structure like that set forth above for the enclosure 102; however, the enclosure 602 includes a top frame 603 including the translucent plastic stretched between portions of the top frame 603, as well as translucent plastic hanging from the top frame 603. That said, the enclosure 602 is not limited to the top frame 603. Indeed, the enclosure 602 can further include a bottom frame framing a patient-interfacing portion like that of the enclosure 102 or even framed ends like the pair of framed ends 112 of the enclosure 102.

The enclosure 602 is configured with an unexpanded state in which the translucent plastic attached to the top frame 603 is folded into the enclosure 602 or bunched up under the top frame 603. The enclosure 602 is also configured with an expanded state in which the translucent plastic attached to the top frame 603 hangs from the top frame 603. A transition from the unexpanded state to the expanded state of the enclosure 602 is effectuated by hanging the enclosure 602 on a boom or the like by the top frame 603 using a cord.

While not shown, the enclosure 602 includes one or more features set forth above for the enclosure 102 selected from at least the air inlet 114, the fenestration 110, the one-or-more pairs of long-sleeved gloves 116, the one-or-more closed-ended tubes 117, the one-or-more pockets 118, and the introducing means for introducing procedural equipment or supplies into the sterile environment inside of the enclosure 602.

### Systems for establishing a sterile environment

FIG. 1 illustrates the system 100 including the enclosure 102 for establishing a sterile environment in accordance with some embodiments. While the system 100 set forth below is described with reference to the enclosure 102, it should be understood the system 100 can alternatively include the enclosure 502 or 602.

As shown, the system 100 includes the enclosure 102 for placement over the patient P, as well as sterile procedural equipment 132 or supplies 134 for the medical procedure in the sterile environment inside of the enclosure 102.

The enclosure 102 is primarily described in the foregoing section.

The procedural equipment 132 or supplies 134 for the medical procedure can be selected for any medical procedure of a number of medical procedures such as a medical procedure for placing a central venous catheter ("CVC"), a peripherally inserted central catheter ("PICC"), a medical port, or the like. With respect to the procedural equipment 132, the procedural equipment 132 can include without limitation one or more puncturing devices, one or more guidewires selected from access guidewires, maneuver guidewires, and rail guidewires, one or more catheters (e.g., a CVC, a PICC, etc.), one or more syringes, or a combination thereof. With respect to the supplies 134, the supplies 134 can include without limitation one or more packages of an antiseptic (e.g., iodopovidone), saline, or a medicament, one or more swabs, gauze, or a combination thereof.

The system 100 can further include packaging such as a bag or pouch, additional supplies such as one or more pairs of gloves or those selected from the supplies 134 for the medical procedure, instructions for use, manufacturing information, or the like. For example, the enclosure 102 including the procedural equipment 132 or supplies 134 inside the enclosure 102 can be disposed in the bag or pouch in the unexpanded state of the enclosure 102. In addition, the additional supplies such as the one-or-more pairs of gloves, one or more packages of antiseptic, or the like can be disposed in the bag or pouch along with the enclosure 102 for independently removing the additional supplies to prepare the patient P and the area or interest for the medical procedure before placing the enclosure 102 and the fenestration 110 thereof over the area of interest. The bag or pouch can be sterilized with ethylene-oxide gas and sealed with the enclosure 102 and the additional supplies, thereby forming the system 100 in accordance with some embodiments. Such a system can be shipped or stored until ready to use.

Advantageously, the system 100 obviates a need for a clinician performing a medical procedure with the system 100 to don protective equipment such as a gown or mask or even use one or more drapes to set-up a sterile field. As such, the system 100 saves time and money, as well decrease the possibility for compromising a sterile field.

### Methods

Methods of the system 100 include methods for establishing a sterile environment over the patient P for a medical procedure.

A method for establishing a sterile environment over the patientP for a medical procedure includes at least a placing step, an expanding step, and a working step in association with the system 100 as set forth in more detail below.

The placing step includes placing the enclosure 102 over an appendage of the patient P or the main body of the patient P such that the fenestration 110 of the patient-interfacing portion 106 of the barrier is directly over an area of interest of the patient P. When the support system 108 is coupled to the patient-interfacing portion 106 of the barrier, the placing step also includes placing the support system 108 such that the fenestration-flanking supports of the support system 108 also flank the appendage or main body of the patient P.

The expanding step includes expanding the expandable portion 104 of the barrier separating the sterile environment inside of the enclosure 102 from the non-sterile environment outside of the enclosure 102 from the unexpanded state to the expanded state. The expanding step can be before or after the placing step.

Expanding the expandable portion 104 of the barrier can include lifting each framed end of the pair of framed ends 112 of the enclosure 102 from the patient-interfacing portion 106 of the barrier in the unexpanded state of the expandable portion 104 of the barrier to form the expanded state thereof. Simultaneously, filtered air can be introduced into the inside of the enclosure 102 with the air inlet 114 fluidly connected to the inside of the enclosure 102. Alternatively, such as in embodiments in which the expandable portion 104 of the barrier is an inflatable bag, but not limited thereto, the expanding step can include merely inflating the expandable portion 104 of the barrier with the filtered air introduced into the inside of the enclosure 102 with the air inlet 114. During the expanding step, any fenestrations (e.g., the fenestration 110) or other openings should remain sealed such that only filtered air is introduced into the inside of the enclosure 102.

As set forth above, introducing the filtered air into the inside of the enclosure 102 with the air inlet 114 can also introduce the filtered air into the support system 108 when the supports of the support system 108 are fluidly connected to the inside of the enclosure 102. In such embodiments, introducing the filtered air into the support system 108 expands the support system from the unexpanded state to the expanded state in concert with the expandable portion 104 of the barrier. That said, the support system 108 can be alternatively configured to expand from the unexpanded state to the expanded state independent of the expandable portion 104 of the barrier through one or more inlets exclusive to the support system 108. When the enclosure is so configured, the method can include a second expanding step before or after the foregoing expanding step of expanding the supports of the support system 108 from the unexpanded state to the expanded state with a same fluid (e.g., filtered air) or a different fluid (e.g., water) than that for the expandable portion 104 of the barrier.

The working step includes working in the sterile environment to perform the medical procedure.

The method can further include a preparing step of preparing the area of interest with an antiseptic before the placing step.

The method can further include a first removing step followed by an adhering step as set forth in more detail below. The first removing step can be performed at any time relative to the preparing step, but the adhering step is performed after the preparing step in order to maintain sterility.

The first removing step includes removing the outer seal of the fenestration 110 from the patient-interfacing surface of the patient-interfacing portion 106 of the barrier. The first removing step exposes the perimeter around the fenestration 110 having the adhesive on the patient-interfacing surface.

The adhering step includes adhering the patient-interfacing portion 106 of the barrier to the patient P by way of the adhesive exposed during the first removing step such that the perimeter of the fenestration 110 is around the area of interest.

The method can further include an inserting step of inserting at least one hand into a glove of the one-or-more pairs of long-sleeved gloves 116 integrated into the expandable portion 104 of the barrier followed by a second removing step of removing the inner seal of the fenestration 110 from the inner surface of the patient-interfacing portion 106 of the barrier. The second removing step should be after adhering the patient-interfacing portion 106 of the barrier to the patient to maintain the sterile environment over the patient P.

While some particular embodiments have been disclosed herein, and while the particular embodiments have been disclosed in some detail, it is not the intention for the particular embodiments to limit the scope of the concepts provided herein. Accordingly, departures may be made from the particular embodiments disclosed herein without departing from the scope of the appended claims.

## Claims

1. An enclosure (102, 502, 602) for establishing a sterile environment over a patient for a medical procedure, comprising:
an expandable portion (104) of a barrier separating the sterile environment inside of the enclosure (102, 502, 602) from a non-sterile environment outside of the enclosure (102, 502, 602), the expandable portion (104) of the barrier configured to expand from an unexpanded state to an expanded state for working within the sterile environment;
a patient-interfacing portion (106) of the barrier including a fenestration (110) configured for placement directly over an area of interest of a patient, the fenestration (110) having a sealed state and an unsealed state for access to the area of interest from within the sterile environment;
**characterized in that** it further comprises
a support system (108) coupled to the patient-interfacing portion (106) of the barrier flanking the fenestration (110), the support system (108) configured to support the enclosure (102, 502, 602) on a surface when placed over an appendage of the patient or a main body of the patient positioned in any one of a number of anatomical positions on the surface.

2. The enclosure of claim 1, wherein the support system (108) is configured to expand from an unexpanded state to an expanded state in concert with the expandable portion (104) of the barrier.

3. The enclosure of claim 1, wherein the support system (108) is configured to expand from an unexpanded state to an expanded state independent of the expandable portion (104) of the barrier.

4. The enclosure of any claim of claims 1-3, further comprising a pair of framed ends (112) of the enclosure (102) configured to support the expandable portion (104) of the barrier, each framed end of the pair of framed ends (112) configured to lie down on the patient-interfacing portion (106) of the barrier in the unexpanded state of the barrier and extend vertically from the patient-interfacing portion (106) of the barrier in the expanded state of the barrier.

5. The enclosure of any claim of claims 1-4, further comprising an air inlet (114) fluidly connected to the inside of the enclosure (102) configured for introducing filtered air thereto for inflating the expandable portion (104) of the barrier from the unexpanded state to the expanded state or maintaining a greater pressure inside of the enclosure (102) than outside of the enclosure (102);
optionally wherein the enclosure (102) further comprises an inline high-efficiency particulate-arresting ("HEPA") air filter including an optional antimicrobial coating on a filter medium of the HEPA air filter, the HEPA air filter configured to prevent microbial contamination of the sterile environment inside of the enclosure (102).

6. The enclosure of any claim of claims 1-5, wherein the fenestration (110) includes an outer seal over a patient-interfacing surface of the patient-interfacing portion (106) of the barrier in the sealed state of the fenestration (110);
optionally wherein a perimeter of the patient-interfacing surface of the patient-interfacing portion (106) of the barrier around the fenestration (110) includes an adhesive configured to adhere the patient-interfacing portion (106) of the barrier to the patient with the perimeter of the fenestration (110) around the area of interest subsequent to sterilization thereof, the outer seal of the fenestration (110) configured to protect and maintain an integrity of the adhesive prior to use.

7. The enclosure of claim 6, wherein the fenestration (110) includes an inner seal over an inner surface of the patient-interfacing portion (106) of the barrier in the sealed state of the fenestration (110), the inner seal of the fenestration (110) configured to be removed subsequent to adhering the patient-interfacing portion (106) of the barrier to the patient, thereby maintaining the sterile environment over the patient.

8. The enclosure of any claim of claims 1-7,
further comprising one or more pairs of long-sleeved gloves (116) integrated into the expandable portion(104) of the barrier configured to be worn by a clinician when performing the medical procedure within the sterile environment; and/or
further comprising one or more closed-ended tubes (117) integrated into the expandable portion (104) of the barrier configured to accept insertion of a probe for use during the medical procedure within the sterile environment; and/or
further comprising one or more pockets (118) integrated into the expandable portion (104) of the barrier configured to hold procedural equipment or supplies for the medical procedure, the one-or-more pockets (118) inside of the enclosure (102), outside of the enclosure (102), or a combination thereof; and/or
further comprising an introducing means for introducing additional procedural equipment or supplies into the sterile environment inside of the enclosure (102) from the non-sterile environment outside of the enclosure (102).

9. A system for establishing a sterile environment over a patient for a medical procedure, comprising:
an enclosure (102, 502, 602) for placement over a patient including:
an expandable portion (104) of a barrier separating the sterile environment inside of the enclosure (102, 502, 602) from a non-sterile environment outside of the enclosure (102, 502, 602), the expandable portion (104) of the barrier configured to expand from an unexpanded state to an expanded state for working within the sterile environment;
a patient-interfacing portion (106) of the barrier including a fenestration (110) configured for placement directly over an area of interest of a patient, the fenestration (110) having a sealed state and an unsealed state for access to the area of interest from within the sterile environment; and
procedural equipment or supplies for the medical procedure in the sterile environment inside of the enclosure (102, 502, 602);
**characterized in that** it further comprises
a support system (108) coupled to the patient-interfacing portion (106) of the barrier flanking the fenestration (110), the support system (108) configured to support the enclosure (102, 502, 602) on a surface when placed over an appendage of the patient or a main body of the patient positioned in any one of a number of anatomical positions on the surface.

10. The system of claim 9, wherein the fenestration (110) includes an outer seal over a patient-interfacing surface of the patient-interfacing portion (106) of the barrier in the sealed state of the fenestration (110);
optionally wherein a perimeter of the patient-interfacing surface of the patient-interfacing portion (106) of the barrier around the fenestration (110) includes an adhesive configured to adhere the patient-interfacing portion (106) of the barrier to the patient with the perimeter of the fenestration (110) around the area of interest subsequent to sterilization thereof, the outer seal of the fenestration (110) configured to protect and maintain an integrity of the adhesive prior to use.

11. The system of claim 10, wherein the fenestration (110) includes an inner seal over an inner surface of the patient-interfacing portion (106) of the barrier in the sealed state of the fenestration (110), the inner seal of the fenestration (110) configured to be removed subsequent to adhering the patient-interfacing portion (106) of the barrier to the patient, thereby maintaining the sterile environment over the patient.

12. The system of any claim of claims 9-11,
wherein the procedural equipment includes one or more puncturing devices, one or more guidewires selected from access guidewires, maneuver guidewires, and rail guidewires, one or more catheters, one or more syringes, or a combination thereof; and/or
wherein the supplies include one or packages of an antiseptic, saline, or a medicament, one or more swabs, gauze, one or more drapes, or a combination thereof.

## Patentansprüche

1. Umhüllung (102, 502, 602) zur Herstellung einer sterilen Umgebung über einem Patienten für einen medizinischen Eingriff, umfassend:
einen ausdehnbaren Abschnitt (104) einer Barriere, der die sterile Umgebung innerhalb der Umhüllung (102, 502, 602) von einer nicht sterilen Umgebung außerhalb der Umhüllung (102, 502, 602) trennt, wobei der ausdehnbare Abschnitt (104) der Barriere so ausgebildet ist, dass er sich von einem nicht ausgedehnten Zustand in einen ausgedehnten Zustand ausdehnt, um innerhalb der sterilen Umgebung zu arbeiten;
einen Patientenschnittstellenabschnitt (106) der Barriere, der eine Fensterung (110) einschließt, die so ausgebildet ist, dass sie direkt über einem Interessenbereich eines Patienten platziert werden kann, wobei die Fensterung (110) einen abgedichteten Zustand und einen nicht abgedichteten Zustand aufweist, um von innerhalb der sterilen Umgebung einen Zugang zu dem Interessenbereich zu ermöglichen;
**dadurch gekennzeichnet, dass** sie weiter umfasst
ein Stützsystem (108), das mit dem Patientenschnittstellenabschnitt (106) der Barriere angrenzend an die Fensterung (110) gekoppelt ist, wobei das Stützsystem (108) so ausgebildet ist, dass es die Umhüllung (102, 502, 602) auf einer Oberfläche stützt, wenn diese über einem Körperglied des Patienten oder einem Hauptkörper des Patienten platziert ist, der sich in einer beliebigen einer Anzahl von anatomischen Positionen auf der Oberfläche befindet.

2. Umhüllung nach Anspruch 1, wobei das Stützsystem (108) so ausgebildet ist, dass es sich gemeinsam mit dem ausdehnbaren Abschnitt (104) der Barriere von einem nicht ausgedehnten Zustand in einen ausgedehnten Zustand ausdehnt.

3. Umhüllung nach Anspruch 1, wobei das Stützsystem (108) so ausgebildet ist, dass es sich unabhängig von dem ausdehnbaren Abschnitt (104) der Barriere von einem nicht ausgedehnten Zustand in einen ausgedehnten Zustand ausdehnt.

4. Umhüllung nach einem Anspruch der Ansprüche 1-3, weiter umfassend ein Paar gerahmter Enden (112) der Umhüllung (102), die so ausgebildet sind, dass sie den ausdehnbaren Abschnitt (104) der Barriere stützen, wobei jedes gerahmte Ende des Paars gerahmter Enden (112) so ausgebildet ist, dass es in dem nicht ausgedehnten Zustand der Barriere auf dem Patientenschnittstellenabschnitt (106) der Barriere aufliegt und sich in dem ausgedehnten Zustand der Barriere vertikal von dem Patientenschnittstellenabschnitt (106) der Barriere erstreckt.

5. Umhüllung nach einem Anspruch der Ansprüche 1-4, weiter umfassend einen Lufteinlass (114), der strömungstechnisch mit dem Inneren der Umhüllung (102) verbunden ist und so ausgebildet ist, dass er gefilterte Luft in diese einleitet, um den ausdehnbaren Abschnitt (104) der Barriere von dem nicht ausgedehnten Zustand in den ausgedehnten Zustand aufzublasen oder einen höheren Druck im Inneren der Umhüllung (102) als außerhalb der Umhüllung (102) aufrechtzuerhalten;
wobei die Umhüllung (102) optional weiter einen Inline-Hocheffizienz-Partikel-Zurückhaltungs- ("HEPA") Luftfilter umfasst, der eine optionale antimikrobielle Beschichtung auf einem Filtermedium des HEPA-Luftfilters einschließt, wobei der HEPA-Luftfilter so ausgebildet ist, dass er eine mikrobielle Kontamination der sterilen Umgebung innerhalb der Umhüllung (102) verhindert.

6. Umhüllung nach einem Anspruch der Ansprüche 1-5, wobei die Fensterung (110) in dem abgedichteten Zustand der Fensterung (110) eine äußere Dichtung über einer Patientenschnittstellenoberfläche des Patientenschnittstellenabschnitts (106) der Barriere einschließt;
wobei optional ein Umfang der Patientenschnittstellenoberfläche des Patientenschnittstellenabschnitts (106) der Barriere um die Fensterung (110) herum einen Klebstoff einschließt, der so ausgebildet ist, dass er den Patientenschnittstellenabschnitt (106) der Barriere nach deren Sterilisation an den Patienten anbringt, wobei der Umfang der Fensterung (110) um den Interessenbereich herum angeordnet ist, wobei die äußere Dichtung der Fensterung (110) so ausgebildet ist, dass sie den Klebstoff vor der Verwendung schützt und dessen Unversehrtheit aufrechterhält.

7. Umhüllung nach Anspruch 6, wobei die Fensterung (110) in dem abgedichteten Zustand der Fensterung (110) eine innere Dichtung über einer Innenfläche des Patientenschnittstellenabschnitts (106) der Barriere einschließt, wobei die innere Dichtung der Fensterung (110) so ausgebildet ist, dass sie nach dem Anbringen des Patientenschnittstellenabschnitts (106) der Barriere an dem Patienten entfernt wird, wodurch die sterile Umgebung über dem Patienten aufrechterhalten wird.

8. Umhüllung nach einem Anspruch der Ansprüche 1-7,
weiter umfassend ein oder mehrere Paare langärmeliger Handschuhe (116), die in den ausdehnbaren Abschnitt (104) der Barriere integriert sind und so ausgebildet sind, dass sie von einem medizinischen Fachpersonal bei der Durchführung des medizinischen Eingriffs innerhalb der sterilen Umgebung getragen werden können; und/oder
weiter umfassend ein oder mehrere geschlossene Röhren (117), die in den ausdehnbaren Abschnitt (104) der Barriere integriert sind und so ausgebildet sind, dass sie das Einführen einer Sonde zur Verwendung während des medizinischen Eingriffs innerhalb der sterilen Umgebung zulassen; und/oder
weiter umfassend eine oder mehrere Taschen (118), die in den ausdehnbaren Abschnitt (104) der Barriere integriert sind und so ausgebildet sind, dass sie Eingriffsausrüstung oder Zubehör für den medizinischen Eingriff halten, wobei sich die eine oder die mehreren Taschen (118) innerhalb der Umhüllung (102), außerhalb der Umhüllung (102) oder in einer Kombination davon befinden; und/oder
weiter umfassend ein Einführmittel zum Einführen von zusätzlicher Eingriffsausrüstung oder Zubehör in die sterile Umgebung innerhalb der Umhüllung (102) aus der nicht sterilen Umgebung außerhalb der Umhüllung (102).

9. System zur Herstellung einer sterilen Umgebung über einem Patienten für einen medizinischen Eingriff, umfassend:
eine Umhüllung (102, 502, 602) zur Platzierung über einem Patienten, einschließlich:
eines ausdehnbaren Abschnitts (104) einer Barriere, der die sterile Umgebung innerhalb der Umhüllung (102, 502, 602) von einer nicht sterilen Umgebung außerhalb der Umhüllung (102, 502, 602) trennt, wobei der ausdehnbare Abschnitt (104) der Barriere so ausgebildet ist, dass er sich von einem nicht ausgedehnten Zustand in einen ausgedehnten Zustand ausdehnt, um innerhalb der sterilen Umgebung zu arbeiten;
eines Patientenschnittstellenabschnitts (106) der Barriere, der eine Fensterung (110) einschließt, die so ausgebildet ist, dass sie direkt über einem Interessenbereich eines Patienten platziert werden kann, wobei die Fensterung (110) einen abgedichteten Zustand und einen nicht abgedichteten Zustand aufweist, um von innerhalb der sterilen Umgebung einen Zugang zu dem Interessenbereich zu ermöglichen; und
Eingriffsausrüstung oder Zubehör für den medizinischen Eingriff in der sterilen Umgebung innerhalb der Umhüllung (102, 502, 602);
**dadurch gekennzeichnet, dass** sie weiter umfasst
ein Stützsystem (108), das mit dem Patientenschnittstellenabschnitt (106) der Barriere angrenzend an die Fensterung (110) gekoppelt ist, wobei das Stützsystem (108) so ausgebildet ist, dass es die Umhüllung (102, 502, 602) auf einer Oberfläche stützt, wenn diese über einem Körperglied des Patienten oder einem Hauptkörper des Patienten platziert ist, der sich in einer beliebigen einer Anzahl von anatomischen Positionen auf der Oberfläche befindet.

10. System nach Anspruch 9, wobei die Fensterung (110) in dem abgedichteten Zustand der Fensterung (110) eine äußere Dichtung über einer Patientenschnittstellenoberfläche des Patientenschnittstellenabschnitts (106) der Barriere einschließt;
wobei optional ein Umfang der Patientenschnittstellenoberfläche des Patientenschnittstellenabschnitts (106) der Barriere um die Fensterung (110) herum einen Klebstoff einschließt, der so ausgebildet ist, dass er den Patientenschnittstellenabschnitt (106) der Barriere nach deren Sterilisation an den Patienten anbringt, wobei der Umfang der Fensterung (110) um den Interessenbereich herum angeordnet ist, wobei die äußere Dichtung der Fensterung (110) so ausgebildet ist, dass sie den Klebstoff vor der Verwendung schützt und dessen Unversehrtheit aufrechterhält.

11. System nach Anspruch 10, wobei die Fensterung (110) in dem abgedichteten Zustand der Fensterung (110) eine innere Dichtung über einer Innenfläche des Patientenschnittstellenabschnitts (106) der Barriere einschließt, wobei die innere Dichtung der Fensterung (110) so ausgebildet ist, dass sie nach dem Anbringen des Patientenschnittstellenabschnitts (106) der Barriere an dem Patienten entfernt wird, wodurch die sterile Umgebung über dem Patienten aufrechterhalten wird.

12. System nach einem Anspruch der Ansprüche 9-11,
wobei die Eingriffsausrüstung eine oder mehrere Punktionsvorrichtungen, einen oder mehrere Führungsdrähte, ausgewählt aus Zugangsführungsdrähten, Manövrierführungsdrähten und Schienenführungsdrähten, einen oder mehrere Katheter, eine oder mehrere Spritzen oder eine Kombination davon einschließt; und/oder
wobei das Zubehör eine oder mehrere Packungen eines Antiseptikums, einer Kochsalzlösung oder eines Medikaments, einen oder mehrere Tupfer, Mull, ein oder mehrere Abdecktücher oder eine Kombination davon einschließt.

## Revendications

1. Enceinte (102, 502, 602) pour établir un environnement stérile sur un patient pour une intervention médicale, comprenant :
une partie extensible (104) d'une barrière séparant l'environnement stérile à l'intérieur de l'enceinte (102, 502, 602) d'un environnement non stérile à l'extérieur de l'enceinte (102, 502, 602), la partie extensible (104) de la barrière étant configurée pour se dilater d'un état non dilaté à un état dilaté pour travailler à l'intérieur de l'environnement stérile ;
une partie (106) d'interface avec le patient de la barrière incluant une fenestration (110) configurée pour être placée directement sur une zone d'intérêt d'un patient, la fenestration (110) présentant un état scellé et un état non scellé pour l'accès à la zone d'intérêt depuis l'intérieur de l'environnement stérile ;
**caractérisée en ce qu'**elle comprend en outre
un système de support (108) couplé à la partie (106) d'interface avec le patient de la barrière, encadrant la fenestration (110), le système de support (108) étant configuré pour supporter l'enceinte (102, 502, 602) sur une surface lorsqu'elle est placée sur un appendice du patient ou un corps principal du patient positionné dans l'une quelconque d'un certain nombre de positions anatomiques sur la surface.

2. Enceinte selon la revendication 1, dans laquelle le système de support (108) est configuré pour se dilater d'un état non dilaté à un état dilaté de concert avec la partie extensible (104) de la barrière.

3. Enceinte selon la revendication 1, dans laquelle le système de support (108) est configuré pour se dilater d'un état non dilaté à un état dilaté indépendamment de la partie extensible (104) de la barrière.

4. Enceinte selon l'une quelconque des revendications 1 à 3, comprenant en outre une paire d'extrémités à cadre (112) de l'enceinte (102) configurées pour supporter la partie extensible (104) de la barrière, chaque extrémité à cadre de la paire d'extrémités à cadre (112) étant configurée pour reposer sur la partie (106) d'interface avec le patient de la barrière dans l'état non dilaté de la barrière et s'étendre verticalement depuis la partie (106) d'interface avec le patient de la barrière dans l'état dilaté de la barrière.

5. Enceinte selon l'une quelconque des revendications 1 à 4, comprenant en outre une entrée d'air (114) en communication fluidique avec l'intérieur de l'enceinte (102), configurée pour y introduire de l'air filtré afin de gonfler la partie extensible (104) de la barrière depuis l'état non dilaté jusqu'à l'état dilaté ou de maintenir une pression plus élevée à l'intérieur de l'enceinte (102) qu'à l'extérieur de l'enceinte (102) ;
éventuellement dans laquelle l'enceinte (102) comprend en outre un filtre à air à particules à haute efficacité (« HEPA ») en ligne incluant un revêtement antimicrobien facultatif sur un milieu filtrant du filtre à air HEPA, le filtre à air HEPA étant configuré pour empêcher une contamination microbienne de l'environnement stérile à l'intérieur de l'enceinte (102).

6. Enceinte selon l'une quelconque des revendications 1 à 5, dans laquelle la fenestration (110) inclut un joint externe sur une surface d'interface avec le patient de la partie (106) d'interface avec le patient de la barrière dans l'état scellé de la fenestration (110) ;
éventuellement dans laquelle un périmètre de la surface d'interface avec le patient de la partie (106) d'interface avec le patient de la barrière autour de la fenestration (110) inclut un adhésif configuré pour faire adhérer la partie (106) d'interface avec le patient de la barrière au patient avec le périmètre de la fenestration (110) autour de la zone d'intérêt postérieurement à la stérilisation de celle-ci, le joint externe de la fenestration (110) étant configuré pour protéger et maintenir une intégrité de l'adhésif avant utilisation.

7. Enceinte selon la revendication 6, dans laquelle la fenestration (110) inclut un joint interne sur une surface interne de la partie (106) d'interface avec le patient de la barrière dans l'état scellé de la fenestration (110), le joint interne de la fenestration (110) étant configuré pour être retiré postérieurement à l'adhérence de la partie (106) d'interface avec le patient de la barrière au patient, maintenant ainsi l'environnement stérile sur le patient.

8. Enceinte selon l'une quelconque des revendications 1 à 7,
comprenant en outre une ou plusieurs paires de gants à longues manches (116) intégrées dans la partie extensible (104) de la barrière, configurées pour être portées par un clinicien lors de l'exécution de l'intervention médicale à l'intérieur de l'environnement stérile ; et/ou
comprenant en outre un ou plusieurs tubes (117) fermés à une extrémité intégrés dans la partie extensible (104) de la barrière, configurés pour accepter l'insertion d'une sonde destinée à être utilisée pendant l'intervention médicale à l'intérieur de l'environnement stérile ; et/ou
comprenant en outre une ou plusieurs poches (118) intégrées dans la partie extensible (104) de la barrière, configurées pour contenir un équipement d'intervention ou des fournitures pour l'intervention médicale, la une ou les plusieurs poches (118) étant à l'intérieur de l'enceinte (102), à l'extérieur de l'enceinte (102), ou une combinaison de ceux-ci ; et/ou
comprenant en outre un moyen d'introduction pour introduire un équipement d'intervention supplémentaire ou des fournitures supplémentaires dans l'environnement stérile à l'intérieur de l'enceinte (102) à partir de l'environnement non stérile à l'extérieur de l'enceinte (102).

9. Système pour établir un environnement stérile sur un patient pour une intervention médicale, comprenant :
une enceinte (102, 502, 602) destinée à être placée sur un patient, incluant :
une partie extensible (104) d'une barrière séparant l'environnement stérile à l'intérieur de l'enceinte (102, 502, 602) d'un environnement non stérile à l'extérieur de l'enceinte (102, 502, 602), la partie extensible (104) de la barrière étant configurée pour se dilater d'un état non dilaté à un état dilaté pour travailler à l'intérieur de l'environnement stérile ;
une partie (106) d'interface avec le patient de la barrière incluant une fenestration (110) configurée pour être placée directement sur une zone d'intérêt d'un patient, la fenestration (110) présentant un état scellé et un état non scellé pour l'accès à la zone d'intérêt depuis l'intérieur de l'environnement stérile ; et
un équipement d'intervention ou des fournitures pour l'intervention médicale dans l'environnement stérile à l'intérieur de l'enceinte (102, 502, 602) ;
**caractérisé en ce qu'**il comprend en outre
un système de support (108) couplé à la partie (106) d'interface avec le patient de la barrière, encadrant la fenestration (110), le système de support (108) étant configuré pour supporter l'enceinte (102, 502, 602) sur une surface lorsqu'elle est placée sur un appendice du patient ou un corps principal du patient positionné dans l'une quelconque d'un certain nombre de positions anatomiques sur la surface.

10. Système selon la revendication 9, dans lequel la fenestration (110) inclut un joint externe sur une surface d'interface avec le patient de la partie (106) d'interface avec le patient de la barrière dans l'état scellé de la fenestration (110) ;
éventuellement dans lequel un périmètre de la surface d'interface avec le patient de la partie (106) d'interface avec le patient de la barrière autour de la fenestration (110) inclut un adhésif configuré pour faire adhérer la partie (106) d'interface avec le patient de la barrière au patient avec le périmètre de la fenestration (110) autour de la zone d'intérêt postérieurement à la stérilisation de celle-ci, le joint externe de la fenestration (110) étant configuré pour protéger et maintenir une intégrité de l'adhésif avant utilisation.

11. Système selon la revendication 10, dans lequel la fenestration (110) inclut un joint interne sur une surface interne de la partie (106) d'interface avec le patient de la barrière dans l'état scellé de la fenestration (110), le joint interne de la fenestration (110) étant configuré pour être retiré postérieurement à l'adhérence de la partie (106) d'interface avec le patient de la barrière au patient, maintenant ainsi l'environnement stérile sur le patient.

12. Système selon l'une quelconque des revendications 9 à 11,
dans lequel l'équipement d'intervention inclut un ou plusieurs dispositifs de ponction, un ou plusieurs fils-guides choisis parmi des fils-guides d'accès, des fils-guides de manœuvre et des fils-guides de rail, un ou plusieurs cathéters, une ou plusieurs seringues, ou une combinaison de ceux-ci ; et/ou
dans lequel les fournitures incluent un ou plusieurs conditionnements d'un antiseptique, d'une solution saline ou d'un médicament, un ou plusieurs écouvillons, de la gaze, un ou plusieurs champs, ou une combinaison de ceux-ci.
